Europäisches Patentamt

European Patent Office

Office européen des brevets

(11) Numéro de publication : **0 097 573**
**B1**

## (12) FASCICULE DE BREVET EUROPÉEN

(45) Date de publication du fascicule du brevet :
10.09.86

(21) Numéro de dépôt : 83401182.7

(22) Date de dépôt : 09.06.83

(51) Int. Cl.⁴ : **G 01 N 33/543, B 01 L 3/14**

(54) **Dispositif pour le dosage d'un composé biologique, son procédé de fabrication et son utilisation dans un procédé de dosage.**

(30) Priorité : 15.06.82 FR 8210415

(43) Date de publication de la demande :
04.01.84 Bulletin 84/01

(45) Mention de la délivrance du brevet :
10.09.86 Bulletin 86/37

(84) Etats contractants désignés :
BE CH DE GB IT LI LU NL SE

(56) Documents cités :
EP-A- 0 024 174
FR-A- 2 458 070
GB-A- 2 015 158
US-A- 4 317 810

(73) Titulaire : **Compagnie ORIS INDUSTRIE SA**
**31-33, rue de la Fédération**
**F-75015 Paris (FR)**

(72) Inventeur : **Gaussens, Gilbert**
**11, rue Jean Brunet**
**F-92190 Meudon (FR)**
Inventeur : **Marchand, Joseph**
**14, Le Colombier**
**F-30130 Pont Saint Esprit (FR)**
Inventeur : **Noaillac, Jean Roch**
**3 Clos Perrault**
**F-91200 Athis Mons (FR)**
**L'autre inventeur a renoncé à sa désignation**

(74) Mandataire : **Mongrédien, André et al**
**c/o BREVATOME 25, rue de Ponthieu**
**F-75008 Paris (FR)**

# 0 097 573

## Description

L'invention a pour objet un dispositif pour le dosage d'un composé biologique, son procédé de fabrication et son utilisation dans un procédé de dosage par un traceur non radioactif.

Ce dispositif peut s'appliquer à l'analyse de solutions contenant des composés biologiques tels que des antigènes, des anticorps, des hormones, des vitamines, des protéines, et des enzymes en utilisant les réactions antigène-anticorps, les réactions hormone-protéine, les réactions vitamine-protéine ou les réactions enzyme-substrat. Généralement, dans le cas de la réaction antigène-anticorps, le dosage de ces composés biologiques est effectué, soit par radioimmunologie, soit par immunologie en utilisant un traceur non radioactif.

Dans le cas du dosage d'antigènes ou d'anticorps, la méthode radioimmunologique consiste à étudier la réaction antigène-anticorps à l'aide d'un traceur radioactif obtenu en marquant l'antigène ou l'anticorps par une substance radioactive telle que l'iode 125. Plusieurs modes opératoires sont possibles. On utilise fréquemment la méthode dans laquelle on fait réagir par exemple l'antigène à déterminer avec un excès d'anticorps, puis dans une étape ultérieure, on ajoute de l'anticorps marqué qui se lie à l'antigène se trouvant en quantité limitée par rapport à la quantité totale d'anticorps. On sépare ensuite les complexes antigène-anticorps du reste de la solution et on détermine la radioactivité des complexes antigène-anticorps, ce qui, par comparaison avec une courbe standard, permet de déterminer la quantité d'antigène présente dans l'échantillon. Dans un procédé de ce type, il est donc nécessaire de séparer les complexes antigène-anticorps de la solution et ceci peut être effectué en phase solide par utilisation d'un support en matériau approprié susceptible par exemple, de fixer l'anticorps et le complexe formé ensuite par réaction de l'antigène avec l'anticorps, comme cela est décrit dans la demande de brevet français FR-A-2418457. Dans ce but, on a déjà utilisé des tubes sur la paroi desquels est fixé l'un des réactifs, ou bien des billes ou d'autres pièces chargées de réactif que l'on introduit dans le tube où s'effectuent les réactions.

Dans cette méthode de dosage radioimmunologique, il est donc nécessaire de déterminer la radioactivité des complexes formés et cette mesure peut être effectuée en fin d'opération directement dans le tube où l'on réalise les différentes réactions.

Parmi les méthodes de dosage utilisant un traceur non radioactif, on connaît des méthodes de dosage immunoenzymatique. Dans ce cas, lorsque le composé biologique à doser est un antigène, entre autres techniques, on met en contact l'échantillon contenant l'antigène avec un anticorps spécifique fixé en quantité déterminée sur un support solide de façon à former le complexe antigène-anticorps et à le retenir sur le support, puis on élimine la partie non réactive de l'échantillon, et on met en contact le support sur lequel est fixé le complexe anticorps-antigène avec une solution d'un composé conjugué d'un anticorps spécifique de l'antigène et d'une enzyme de façon à former et à fixer sur le support ce conjugué par l'antigène du précédent complexe entre l'anticorps sur le support et l'antigène ayant réagi. Après formation de ce dernier complexe et élimination de la solution du composé conjugué n'ayant pas réagi, on met en contact le support avec un substrat de l'enzyme du composé conjugué de façon à réaliser une réaction enzymatique que l'on arrête au bout d'un certain temps, puis on mesure la densité optique de la solution pour en déterminer l'activité enzymatique.

Comme précédemment, on peut utiliser des tubes revêtus d'anticorps ou contenant une pièce sur laquelle est fixé l'anticorps pour réaliser les différentes réactions. Cependant, pour mesurer en fin d'opération la densité optique de la solution, il est nécessaire de transvaser le contenu du tube dans la cuve d'un spectrophotomètre, ce qui nécessite des manipulations et des opérations de lavage complémentaires par rapport au dosage radioimmunologique où l'on peut déterminer directement la radioactivité dans le tube où ont été effectuées les différentes réactions.

La présente invention a précisément pour objet un dispositif pour le dosage d'un composé biologique capable de réagir avec un composé antagoniste spécifique dudit composé biologique, qui peut être utilisé en particulier dans les procédés de dosage par traceur non radioactif nécessitant la détermination d'une caractéristique optique.

Selon l'invention, le dispositif pour le dosage d'un composé biologique susceptible de réagir avec un composé antagoniste spécifique dudit composé biologique et comportant une pièce en matériau susceptible de fixer ledit composé biologique ou le composé antagoniste spécifique dudit composé biologique, se caractérise en ce qu'il est constitué par un tube transparent, sensiblement cylindrique de section circulaire, fermé à sa base et comportant successivement depuis sa base une première zone de réaction (A) dans laquelle est engagée et immobilisée ladite pièce, et une seconde zone (B) permettant de déterminer directement les caractéristiques optiques d'une solution présente dans ledit tube, les surfaces interne et externe de la paroi dudit tube étant centrées l'une par rapport à l'autre au moins dans ladite seconde zone (B).

Dans le dispositif de l'invention, la seconde zone que nous dénommerons, ci-après, zone de lecture présente ainsi les qualités optiques nécessaires pour obtenir une bonne reproductibilité et une bonne précision des mesures. En effet, en s'assurant que les surfaces interne et externe de la paroi du tube sont parfaitement centrées, on obtient dans la zone de lecture des épaisseurs de paroi constantes et de ce fait, quelle que soit la position du tube par rapport à un faisceau lumineux, l'absorption du faisceau par la

2

0 097 573

paroi est identique dans tous les cas.

Selon une caractéristique du dispositif de l'invention, la pièce engagée et immobilisée dans la première zone de réaction a la forme d'un manchon cylindrique plein muni d'ailettes longitudinales dont les bords externes sont en contact avec la surface interne de la paroi dudit tube. De préférence, l'extrémité inférieure du manchon est en contact avec le fond dudit tube.

Généralement, la pièce est en matériau élastique insoluble dans les différentes solutions utilisées, par exemple, en polyamide.

Selon une caractéristique préférentielle du dispositif de l'invention, le fond du tube est presque plat, ce qui permet de réaliser dans de meilleures conditions le lavage du tube et de limiter, par ailleurs, les quantités de réactif mises en jeu. En effet, pour obtenir la réaction entre le composé biologique à analyser et le composé fixé sur la pièce engagée et immobilisée dans la zone de réaction du tube, il suffit d'utiliser une quantité de solution permettant l'immersion de la pièce. Lorsque le tube est à fond arrondi, les ailettes ne peuvent être engagées au niveau du fond du tube et de ce fait il reste un volume perdu à la base de celui-ci.

Selon l'invention, le tube est réalisé en un matériau transparent, par exemple en matériau thermoplastique transparent tel que le polystyrène, le polyméthacrylate de méthyle, les polyacryliques ou les copolymères acryliques.

L'invention a également pour objet un procédé de fabrication d'un tube transparent à fond plat sensiblement cylindrique de section circulaire présentant la qualité optique voulue pour être utilisé dans le dispositif de l'invention.

Ce procédé consiste à réaliser ledit tube par moulage par injection d'un matériau thermoplastique en utilisant un moule en plusieurs parties comportant des évents sur sa partie qui correspond à l'empreinte de fond dudit tube et en réalisant l'injection par injection capillaire sous-marine en deux points du moule qui correspondent à deux points diamétralement opposés de la surface externe du tube à mouler.

Avantageusement, la partie du moule qui correspond à l'empreinte de fond dudit tube est constituée par un empilement de pièces disposées les unes à côté des autres avec un faible jeu pour former les évents.

L'invention a encore pour objet un procédé de dosage d'un composé biologique susceptible de réagir avec un composé antagoniste spécifique dudit composé biologique, utilisant le dispositif de l'invention.

Ce procédé de dosage qui comporte plusieurs étapes de réaction et de lavage et une étape de détermination d'une caractéristique optique d'une solution, se caractérise en ce que l'on réalise toutes les étapes dans le dispositif de l'invention et en ce que l'on détermine directement dans ledit tube la caractéristique optique de ladite solution.

Généralement la caractéristique optique à déterminer est la densité optique de la solution. Cependant, on peut aussi utiliser le dispositif de l'invention pour déterminer d'autres caractéristiques optiques, mettant en jeu des phénomènes d'absorption, de diffusion, de transmission ou d'émission de rayonnements, par exemple la luminescence de la solution.

D'autres caractéristiques et avantages de l'invention apparaîtront mieux à la lecture de la description qui suit donnée bien entendu à titre illustratif et non limitatif en référence au dessin annexé sur lequel :

— la figure 1 représente schématiquement en coupe verticale un dispositif de dosage selon l'invention,

— la figure 2 illustre la réalisation d'un tube par moulage par injection selon l'art antérieur,

— la figure 3 illustre la réalisation d'un tube par moulage par injection selon l'invention,

— la figure 4 représente la progression de la matière dans le moule de la figure 3, et

— la figure 5 illustre la réalisation d'un tube par moulage par injection avec une seule alimentation du moule.

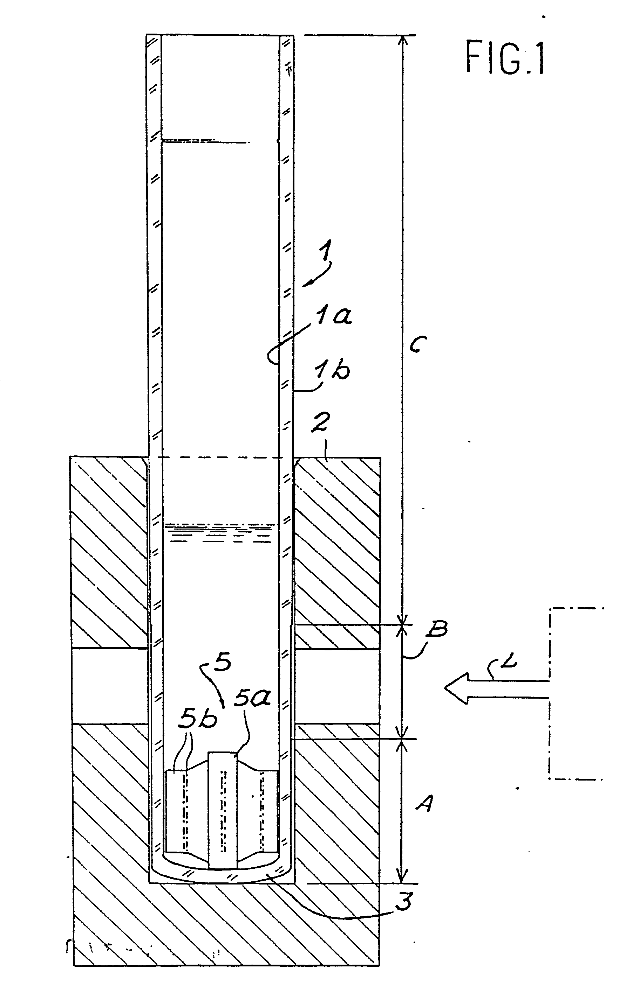

Sur la figure 1, on voit que le dispositif de l'invention est constitué par un tube transparent 1 presque cylindrique de section circulaire qui est fermé à sa base par un fond plat 3. Ce tube comprend successivement depuis sa base une première zone de réaction A dans laquelle est engagée et immobilisée une pièce 5 destinée à fixer le composé biologique ou le composé antagoniste spécifique du composé biologique à doser.

Cette pièce comprend un manchon cylindrique plein 5a dont l'extrémité inférieure repose sur le fond 3 du tube 1 et des ailettes longitudinales 5b dont les bords externes viennent en contact avec la surface interne 1a du tube 1, ce qui permet d'assurer l'immobilisation de la pièce dans le tube puisque cette pièce est réalisée en matériau élastique, par exemple, en polyamide. La hauteur de cette pièce 5 détermine la hauteur de la zone de réaction A du tube 1.

Comme on peut le voir sur le dessin, le fait d'avoir un fond plat 3 permet de limiter les quantités de réactif à utiliser, qui doivent être juste suffisantes pour immerger totalement la pièce 5. Au-dessus de la première zone de réaction A, le tube comporte une seconde zone de lecture B, puis une zone C de hauteur plus importante permettant de réaliser les étapes de lavage. Dans la seconde zone de lecture B, le tube présente une très bonne qualité optique qui est obtenue en particulier grâce au fait que la surface interne 1a et la surface externe 1b de la paroi du tube 1 sont bien centrées l'une par rapport à l'autre, tout au moins dans cette zone. Ainsi, dans cette zone du tube, l'épaisseur e de la paroi est homogène et constante.

3

Le tube 1 est réalisé en matière plastique, par exemple en polyméthacrylate de méthyle et il est obtenu de préférence par moulage par injection selon le procédé de l'invention, car les techniques classiques de moulage des matières thermoplastiques ne permettent pas l'obtention d'un tube à fond plat présentant la qualité optique voulue pour qu'on puisse mesurer directement dans le tube la densité optique d'une solution.

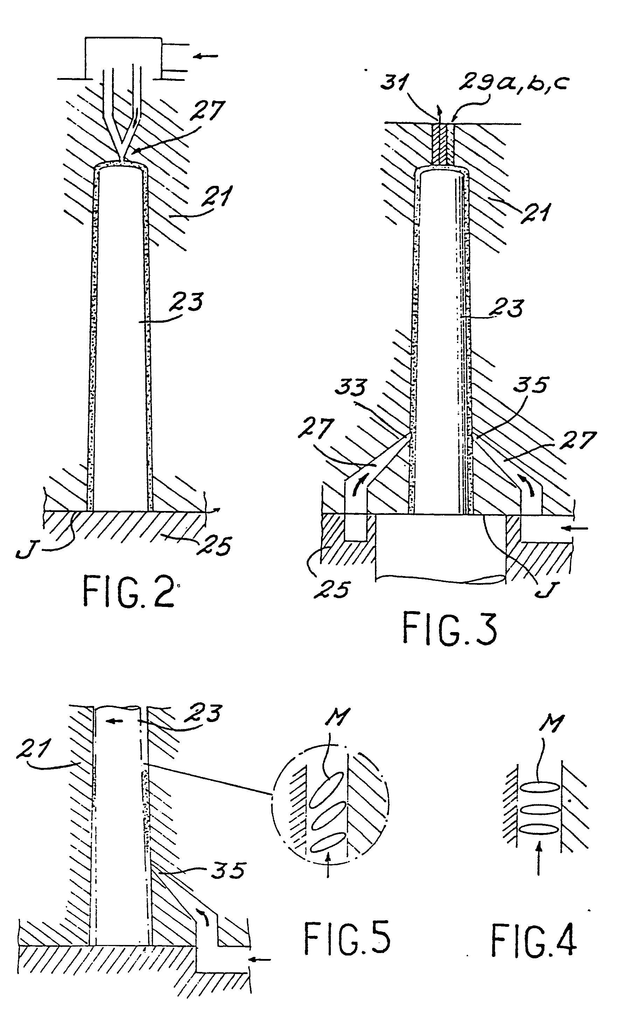

On rappelle que la technique d'injection des matières thermoplastiques consiste à remplir la cavité d'un moule avec une matière en fusion à l'état généralement pâteux et sous pression, afin d'obtenir après refroidissement une pièce de forme voulue. Habituellement, comme on peut le voir sur la figure 2, on réalise des pièces creuses fermées à leur base en utilisant un moule en plusieurs parites comprenant l'empreinte (21) de la surface externe de la pièce, le noyau interne (23) et une pièce (25) permettant l'ouverture du moule en fin d'opération au niveau du plan de joint J.

Dans ce cas, on alimente généralement le moule par le fond (27). Cependant, dans ces conditions, la progression préférentielle de la matière qui devient le plus en plus pâteuse, pousse le noyau 23 qui flambe et se décentre. Ainsi, on obtient un tube dont la surface interne 1a est décentrée par rapport à la surface externe 1b et la qualité optique de ce tube ne permet pas de réaliser des mesures optiques dans de bonnes conditions car les variations d'absorption de la paroi sont élevées lorsqu'on fait tourner le tube autour de son axe et que l'on modifie ainsi sa position par rapport à un faisceau lumineux.

Aussi, selon l'invention, on utilise un procédé d'injection différent qui est illustré sur la figure 3.

Dans ce cas, on utilise comme précédemment un moule en plusieurs parties mais la partie du moule qui correspond à l'empreinte de fond du tube est constituée par un empilement de petites pièces (29a, 29b, 29c, ...) disposées les unes à côté des autres avec un faible jeu pour former des évents (31). Par ailleurs, au lieu de réaliser l'injection par le fond du tube, on réalise une injection capillaire sous-marine, c'est-à-dire une injection en dessous du plan de joint J du moule, en deux points (33, 35) diamètralement opposés de la surface externe du tube à mouler.

Grâce à la présence des évents (31), l'air présent dans le moule est chassé rapidement, ce qui permet d'obtenir une bonne homogénéité de la paroi du tube sans inclusion de bulles d'air. Par ailleurs, le fait d'alimenter le moule en deux points diamétralement opposés permet d'obtenir comme cela est représenté sur la figure 4, une progression homogène de la matière M dans la cavité du moule. Par ailleurs, grâce aux injections latérales, on peut obtenir un bon degré de pureté de la matière thermoplastique sur la paroi du tube car la matière injectée en début d'opération constitue le fond du tube.

En revanche, si l'on réalisait l'injection capillaire sous-marine en un seul point du tube, par exemple en 35, comme représenté sur la figure 5, il se produirait également un flambage du noyau, car la progression de la matière M dans le tube ne serait pas homogène comme on peut le voir sur la figure 5. De ce fait, malgré la présence des évents (31) qui permettent d'évacuer l'air emprisonné dans le moule et d'éviter la formation de bulles et de brûlures de la matière plastique, le tube obtenu n'aurait pas une épaisseur de paroi uniforme.

Pour l'opération de moulage par injection, la température et la pression d'injection sont choisies en fonction de la matière thermoplastique utilisée. Généralement les pressions se situent entre 40 et 200 MPa.

On décrit, ci-après, un exemple de réalisation d'un dispositif de dosage selon l'invention, réalisé en polyméthacrylate de méthyle.

On sèche le polyméthacrylate de méthyle à 80 °C dans une étuve, puis on l'introduit directement dans la machine d'injection où il est porté à une température de 275 °C. Pour l'opération d'injection, on introduit la matière sous une pression d'injection de 110 MPa, dans le moule qui a été porté en début d'opération à une température de 80 °C. Les dimensions du moule sont telles que le tube obtenu ait une hauteur d'environ 70 mm et un diamètre externe de 11,4 mm tout en étant légèrement conique pour faciliter son démoulage. Après refroidissement et solidification du polyméthacrylate de méthyle, on arrache les deux carottes d'injection et on démoule le tube.

Après cette opération, on introduit dans le tube la pièce en forme d'ailette qui est réalisée en polyamide et elle est immobilisée dans le tube par engagement des ailettes sur la surface interne du tube.

Le tube ainsi préparé peut être utilisé pour différents types de dosages, par exemple pour le dosage enzymo-immunologique de l'α-foetoprotéine, pour la détection de l'AgHB$_s$, pour la détection de l'antigène carcino-embryonnaire (CEA) ou pour déterminer le taux d'IgE totales circulant dans les échantillons de sang.

On décrit, ci-après, l'utilisation du dispositif de l'invention pour le dosage immuno-enzymatique de l'α-foetoprotéine AFP qui est une glycoprotéine ayant un poids moléculaire de 70 000, synthétisée par les cellules du foie foetal et du sac vitellin. C'est un constituant majeur du sérum foetal avec un taux maximum atteignant 3 mg/ml vers la 13e semaine et on la trouve dans le liquide amniotique à un taux maximum de 20 à 50 µg/ml, vers la 15e semaine, et dans le sérum maternel, vers la 34e semaine, à un taux de 200 à 500 ng/ml.

On fixe tout d'abord sur l'ailette immobilisée dans le tube une quantité suffisante de l'anticorps anti-AFP obtenu chez le mouton en immergeant l'ailette pendant 18h, à la température ambiante, dans une solution de sérum de mouton anti-AFP humaine. On introduit alors dans le tube 100 µl de l'échantillon contenant l'α-foetoprotéine (AFP). Puis, on complète à 500 µl par une solution contenant des protéines sériques de mouton. On porte ensuite le tube dans un bain-marie dont la température a été ajustée à 37 ± 1 °C, afin de réaliser l'incubation pendant 2h. On vide alors le tube de son contenu et on le lave 2 fois au moyen de 3 ml d'eau distillée. On ajoute alors dans le tube 500 μl du composé conjugué d'anti-AFP couplé à l'Horse radish peroxydase (HRP) et après une légère agitation pour s'assurer que les bulles sont évacuées, on porte le tube dans une étuve maintenue à 37° ± 1 °C et on laisse incuber pendant 1 h 30. Après cette opération, on vide le tube de son contenu et on le lave deux fois par 3 ml d'eau distillée. On ajoute alors dans le tube 500 μl d'une solution-substrat qui a été préparée en introduisant 16 ml de tampon substrat (tampon au citrate contenant 0,02 % de $H_2O_2$) dans un flacon contenant 32 mg de chromogènes OPD (o-phénylène-diamine · 2HCl) à l'état lyophilisé. On laisse incuber le tube pendant 30 min à la température ambiante, à l'abri d'une lumière trop vive, et on arrête alors la réaction enzymatique en ajoutant dans le tube 2 ml d'acide oxalique 1N. On agite modérément puis on introduit les tubes contenant la solution colorée dans un spectrophotomètre pour en déterminer la densité optique à 492 nm, cette mesure étant effectuée dans les deux heures qui suivent l'arrêt de la réaction enzymatique.

En se reportant à la figure 1, on peut voir la disposition du tube 1 dans le spectrophotomètre 2 et l'on voit que le faisceau lumineux L traverse le tube 1 au niveau de la zone de lecture B du tube 1.

Pour vérifier que le tube obtenu présente une bonne homogénéité de paroi, on réalise trois mesures sur le même tube après avoir effectué une rotation de 120 °C par rapport à l'axe du tube pour obtenir 3 positions différentes du tube par rapport au faisceau lumineux. On répète ces opérations avec d'autres échantillons ayant des concentrations différentes en AFP.

Les résultats obtenus sont donnés dans le tableau 1 qui suit :

Tableau 1

| Concentration en AFP (ng/ml) | 1<sup>re</sup> position | 2<sup>e</sup> position | 3<sup>e</sup> position |
|---|---|---|---|
| 0 | 0,096 | 0,102 | 0,096 |
| 3 | 0,129 | 0,134 | 0,131 |
| 12 | 0,216 | 0,217 | 0,216 |
| 40 | 0,598 | 0,598 | 0,599 |
| 120 | 0,958 | 0,960 | 0,960 |

Pour vérifier que ces résultats sont conformes aux résultats obtenus lorsqu'on transfère la solution dans la cuvette du colorimètre, on verse les solutions des tubes dans les cuvettes en polystyrène et on lit les densités optiques sur le même appareil que précédemment. Dans un second temps, on effectue la lecture avec un colorimètre automatique à microcuve en quartz à faces parallèles (colorimètre Seroa-Saitron). Les résultats obtenus sont donnés dans le tableau 2 qui suit.

Tableau 2

| Concentration en AFP (ng/ml) | Transvasement et cuvette en polystyrène | Transvasement par aspiration et microcuve de quartz |
|---|---|---|
| 0 | 0,123 | 0,118 |
| 3 | 0,163 | 0,160 |
| 12 | 0,262 | 0,251 |
| 40 | 0,693 | 0,670 |
| 120 | 1,098 | 1,087 |

Ces résultats montrent un accord suffisant entre les trois mesures, la calibration des appareils et la nature des cuvettes étant les causes principales des faibles différences mesurées. Par ailleurs, on note que le dispositif de l'invention a un bruit de fond faible à 492 nm.

Ainsi, le dispositif de l'invention présente de nombreux avantages pour la réalisation de dosages au moyen de traceurs non radioactis, nécessitant par exemple une mesure de densité optique.

**Revendications**

1. Dispositif pour le dosage d'un composé biologique susceptible de réagir avec un composé antagoniste spécifique dudit composé biologique, et comportant une pièce en matériau susceptible de fixer ledit composé biologique ou le composé antagoniste spécifique dudit composé biologique, caractérisé en ce qu'il est constitué par un tube (1) transparent, sensiblement cylindrique de section circulaire, fermé à sa base et comportant successivement depuis sa base une première zone de réaction

(A) dans laquelle est engagée et immobilisée ladite pièce (5), et une seconde zone (B) permettant de déterminer directement les caractéristiques optiques d'une solution présente dans ledit tube, les surfaces interne (1a) et externe (1b) de la paroi dudit tube étant centrées l'une par rapport à l'autre au moins dans ladite seconde zone (B).

2. Dispositif selon la revendication 1, caractérisé en ce que le fond (3) dudit tube est plat.

3. Dispositif selon l'une quelconque des revendications 1 et 2, caractérisé en ce que ledit tube est réalisé en matériau thermoplastique transparent.

4. Dispositif selon la revendication 3, caractérisé en ce que le matériau transparent est du polyméthacrylate de méthyle.

5. Dispositif selon l'une quelconque des revendications 1 à 4, caractérisé en ce que la pièce (5) engagée et immobilisée dans la première zone de réaction dudit tube, a la forme d'un manchon cylindrique plein (5a) muni d'ailettes longitudinales (5b) dont les bords externes sont en contact avec la surface interne (1a) de la paroi dudit tube.

6. Dispositif selon la revendication 5, caractérisé en ce que l'extrémité inférieure du manchon (5a) est en contact avec le fond (3) dudit tube.

7. Dispositif selon l'une quelconque des revendications 1 à 6, caractérisé en ce que ladite pièce (5) est en matériau élastique.

8. Dispositif selon la revendication 6, caractérisé en ce que ladite pièce (5) est en polyamide.

9. Procédé de fabrication d'un tube à fond plat de section circulaire utilisable dans le dispositif de dosage d'un composé biologique selon l'une quelconque des revendications 2 à 4, caractérisé en ce qu'il consiste à réaliser ledit tube par moulage par injection d'un matériau thermoplastique en utilisant un moule en plusieurs parties (21, 23, 25) comportant des évents (31) sur sa partie qui correspond à l'empreinte de fond dudit tube et en réalisant l'injection par injection capillaire sous-marine en deux points (33, 35) du moule qui correspondent à deux points diamétralement opposés de la surface externe de la paroi du tube à mouler.

10. Procédé selon la revendication 9, caractérisé en ce que la partie du moule qui correspond à l'empreinte de fond dudit tube est constituée par un empilement de pièces (29a, 29b, 29c) disposées les unes à côté des autres avec un faible jeu pour former les évents (31).

11. Procédé de dosage d'un composé biologique, comportant plusieurs étapes de réaction et de lavage et une étape de détermination d'une caractéristique optique d'une solution, caractérisé en ce qu'on réalise toutes les étapes dans le dispositif selon l'une quelconque des revendications 1 à 8, et en ce que l'on détermine directement dans le tube la caractéristique optique de ladite solution.

12. Procédé selon la revendication 11, caractérisé en ce que ladite caractéristique optique est la densité optique.

**Claims**

1. Apparatus for the determination of a biological composition which is capable of reacting with a composition which is a specific antagonist for said biological composition, and comprising a member of a material adapted to fix said biological composition, or the composition which is a specific antagonist for said biological composition, characterised in that it comprises a transparent tube (1) which is substantially cylindrical with circular cross-section, closed at its base, and comprising in succession, beginning at its base, a first reaction zone (A) within which said member (5) is engaged and immobilised, and a second zone (B) permitting direct determination of the optical characteristics of a solution present in said tube, the internal (1a) and external (1b) surfaces of the walls of said tube being centred with respect to one another at least in said second zone (B).

2. Apparatus according to claim 1, characterised in that the bottom (3) of said tube is flat.

3. Apparatus according to either of claims 1 und 2, characterised in that said tube is formed from a transparent thermoplastic material.

4. Apparatus according to claim 3, characterised in that the transparent material is polymethylmethacrylate.

5. Apparatus according to any one of claims 1 to 4, characterised in that the member (5) which is engaged and immobilised in the first reaction zone of said tube, has the form of a solid cylinder (5a) having longitudinal flanges (5b) whose external rims are in contact with the internal surface (1a) of the wall of said tube.

6. Apparatus according to claim 5, characterised in that the lower end of the cylinder (5a) is in contact with the bottom (3) of said tube.

7. Apparatus according to any one of claims 1 to 6, characterised in that said member (5) is formed of elastic material.

8. Apparatus according to claim 6, characterised in that said member (5) is formed of polyamide.

9. Process for manufacture of a tube having a flat bottom and circular cross section utilisable in the apparatus for determination of a biological compostion according to any one of claims 2 to 4, characterised in that it comprises forming said tube by injection moulding of a thermoplastics material employing a multi-part mould (21, 23, 25) comprising risers (31) in the part corresponding to the impression of the bottom of said tube, and producing injection by upwardly-rising capillary injection at two points (33, 35) of the mould corresponding to two diametrically-opposed points on the external surface of the wall of the tube being moulded.

10. Process according to claim 9, characterised in that the part of the mould corresponding to the impression of the bottom of said tube comprises a stack of members (29a, 29b, 29c) located side-by-side, with a small clearance to form the risers (31).

11. Process for the determination of a biological composition, comprising a plurality of reaction and washing steps, and a step involving determination of an optical characteristic of a solution, characterised in that all the steps are carried out in an apparatus according to any one of claims 1 to 8, and in that the optical characteristic of said solution is directly determined in the tube.

12. Process according to claim 11, characterised in that said optical characteristic is the optical density.

**Patentansprüche**

1. Einrichtung zur Bestimmung einer biologischen Verbindung, die geeignet ist, mit einer für diese biologische Verbindung spezifischen gegenwirkenden Verbindung zu reagieren und die einen Körper aus einem Material enthält, das dazu geeignet ist, die genannte biologische Verbindung oder die für diese biologische Verbindung spezifische gegenwirkende Verbindung zu fixieren, dadurch gekennzeichnet, daß sie von einem transparenten Rohr (1) im wesentlichen zylindrischen Querschnitts gebildet ist, das an seiner Basis verschlossen ist und aufeinanderfolgend, von seiner Basis ausgehend, eine erste Reaktionszone (A), in der der genannte Körper (5) gehalten und festgelegt ist, und eine zweite Zone (B) enthält, die es erlaubt, direkt die optischen Eigenschaften einer in dem Rohr enthaltenen Lösung zu bestimmen, wobei die Innenwand (1a) und die Außenwand (1b) des genannten Rohres wenigstens in der genannten zweiten Zone (B) zueinander zentrisch sind.

2. Einrichtung nach Anspruch 1, dadurch gekennzeichnet, daß der Boden (3) des genannten Rohres flach ist.

3. Einrichtung nach einem der Ansprüche 1 und 2, dadurch gekennzeichnet, daß das genannte Rohr aus einem transparenten thermoplastischen Material besteht.

4. Einrichtung nach Anspruch 3, dadurch gekennzeichnet, daß das transparente Material Methylpolymethacrylat ist.

5. Einrichtung nach einem der Ansprüche 1 bis 4, dadurch gekennzeichnet, daß der Körper (5), der in der ersten Reaktionszone des Rohres gehalten und festgelegt ist, die Form einer massiven zylindrischen Muffe (5a) aufweist, die mit längsverlaufenden Flügeln (5b) versehen ist, deren Außenränder die Innenseite (1a) der Wand des genannten Rohres berühren.

6. Einrichtung nach Anspruch 5, dadurch gekennzeichnet, daß das untere Ende der Muffe (5a) den Boden (3) des genannten Rohres berührt.

7. Einrichtung nach einem der Ansprüche 1 bis 6, dadurch gekennzeichnet, daß der Körper (5) aus einem elastischen Material besteht.

8. Einrichtung nach Anspruch 6, dadurch gekennzeichnet, daß der genannte Körper (5) aus Polyamid besteht.

9. Verfahren zum Herstellen eines Rohres mit ebenem Boden und kreisförmigem Querschnitt, das in der Einrichtung zum Bestimmen einer biologischen Verbindung nach einem der Ansprüche 2 bis 4 verwendbar ist, dadurch gekennzeichnet, daß es umfaßt: Herstellen des genannten Rohres durch Spritzgießen eines thermoplastischen Materials unter Verwendung einer mehrteiligen Form (21, 23, 25), die Luftkanäle (31) in jenem Bereich aufweist, der dem Abdruck des Bodens des genannten Rohres entspricht, und wobei das Einspritzen durch untergetauchte Kapillarinjektion an zwei Punkten (33, 35) der Form ausgeführt wird, die zwei diametral gegenüberliegenden Punkten der Außenseite der Wand des zu gießenden Rohres entsprechen.

10. Verfahren nach Anspruch 9, dadurch gekennzeichnet, daß der Teil der Form, der dem Abdruck des Bodens des genannten Rohres entspricht, von einem Stapel von Teilen (29a, 29b, 29c) gebildet ist, die nebeneinander mit geringem Spiel angeordnet sind, um die Luftkanäle (31) auszubilden.

11. Verfahren zum Bestimmen einer biologischen Verbindung mit mehreren Reaktions- und Waschschritten und einem Schritt zur Bestimmung einer optischen Eigenschaft einer Lösung, dadurch gekennzeichnet, daß man alle Schritte in der Einrichtung nach einem der Ansprüche 1 bis 8 ausführt und daß man in dem Rohr direkt die optische Eigenschaft der genannten Lösung bestimmt.

12. Verfahren nach Anspruch 11, dadurch gekennzeichnet, daß die genannte optische Eigenschaft die Schwärzung ist.

FIG.1

0 097 573

1

FIG.2

FIG.3

FIG.5

FIG.4

2